(19) Europäisches Patentamt
European Patent Office
Office européen des brevets

(11) **EP 1 793 342 A2**

(12) **EUROPEAN PATENT APPLICATION**

(43) Date of publication:
**06.06.2007 Bulletin 2007/23**

(51) Int Cl.:
***G06T 3/40*** *(2006.01)*      ***G01S 7/52*** *(2006.01)*

(21) Application number: **06024589.1**

(22) Date of filing: **28.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **02.12.2005 KR 20050116852**

(71) Applicant: **MEDISON CO., LTD.**
**Kangwon-do 250-870 (KR)**

(72) Inventors:
• **Kim, Jung Soo**
**Gangnam-gu,**
**Seoul 135-280 (KR)**
• **Kang, Hak Il**
**Gangnam-gu,**
**Seoul 135-280 (KR)**

(74) Representative: **Lorenz, Markus**
**Lorenz & Kollegen**
**Patent- und Rechtsanwaltskanzlei**
**Alte Ulmer Straße 2**
**89522 Heidenheim (DE)**

(54) **Ultrasound imaging system for displaying an original ultrasound image in a real size**

(57) The present invention relates to an ultrasound imaging system, comprising: an ultrasound image signal providing unit for providing ultrasound image signals corresponding to an ultrasound image having preset horizontal and vertical lengths; an image processing unit for forming a first ultrasound image based on the ultrasound image signals and adjusting a size of the first ultrasound image to thereby form a second ultrasound image; and a display unit including a display region having a plurality of dots arrayed in horizontal and vertical directions, the display region being configured to display the first and second ultrasound images, wherein the image processing unit calculates a conversion value for enlarging or reducing the first ultrasound image based on a horizontal or vertical length of the first ultrasound image and a distance between the neighboring dots, and wherein the image processing unit applies the conversion value to the first ultrasound image to thereby form the second ultrasound image.

**Description**

The present application claims priority from Korean Patent Application No-10-2005-0119852 filed on December 2, 2005, the subject matter of which is incorporated herein by reference.

## BACKGROUND

### 1. Field of the Invention

The present invention generally relates to ultrasound imaging systems, and more particularly to an ultrasound imaging system for displaying an ultrasound image in its original size.

### 2 Background of the Invention

An ultrasound imaging system has become an important and popular diagnostic tool since it has a wide range of applications. Specifically, due to its non-invasive and non-destructive nature, the ultrasound imaging system has been extensively used in the medical profession. Modem high-performance ultrasound imaging systems and techniques are commonly used to produce two or three-dimensional diagnostic images of internal features of an object (e.g., human organs).

The ultrasound imaging system generally uses a wide bandwidth transducer to transmit and receive ultrasound signals. The ultrasound imaging system forms images of human internal tissues by electrically exciting an acoustic transducer element or an array of acoustic-transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which arc amplified and processed to produce ultrasound data for an image of the tissues. The ultrasound imaging system is very important in the medical field since it provides physicians with real-time and high-resolution images of human internal features without the need for invasive observation techniques such as surgery.

Generally, the ultrasound imaging system can zoom in and out an ultrasound image. The ultrasound imaging system also has a measurement function for providing an ultrasound image in which the target object is shown in real size. In a conventional ultrasound imaging system, the target object shown in the ultrasound image is either enlarged or reduced compared to its real size and then displayed on a screen. The real size of the target object, which is shown in the ultrasound image, can be measured by using the measurement function. However, since the target object is not displayed in its real size, the operator cannot recognize the real size of the target object without using the measuring function. Therefore, there is a need for adjusting the size of a displayed ultrasound image to display the target object in its real size, thereby allowing a more instinctive and analytic diagnosis.

## BRIEF DESCRIPTION OF THE DRAWINGS

The present invention may be described in detail with reference to the following drawings in which like reference numerals refer to like elements.

Fig. 1 is a block diagram schematically showing an ultrasound imaging system constructed in accordance with a preferred embodiment of the present invention; and

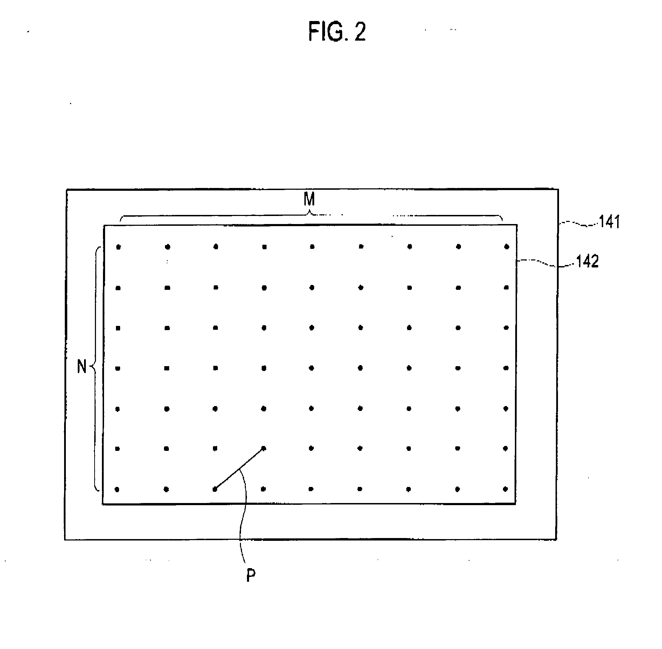

Fig. 2 illustrates an example of a dot array of a screen displaying an ultrasound image.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

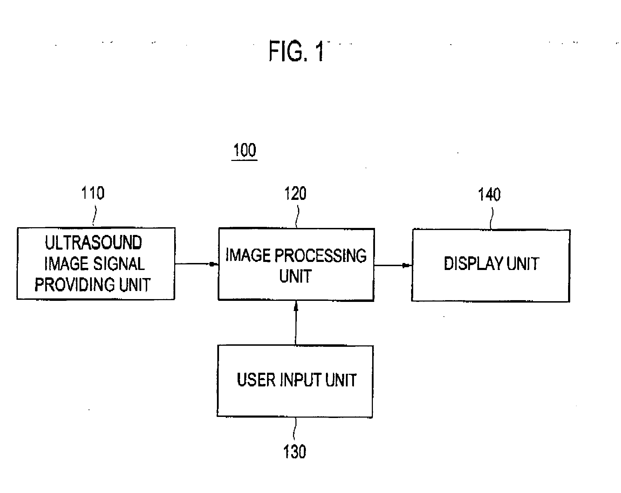

Fig. 1 is a block diagram schematically showing an ultrasound imaging system constructed in accordance with a preferred embodiment of the present invention. As shown in Fig. 1, the ultrasound imaging system 100 includes an ultrasound image signal providing unit 110, an image processing unit 120 for forming an ultrasound image based on ultrasound image signals, a user input unit 130 and a display unit 140 for displaying the ultrasound image formed in the image processing unit 120.

The ultrasound image signal providing unit 110 produces image signals corresponding to an ultrasound image. The image signals are acquired by transmitting ultrasound signals into a predetermined depth of the target object and then receiving ultrasound echo signals. Therefore, the image signal providing unit 110 provides the image signals corresponding to a region defined by a preset depth. That is, the image signal providing unit 110 provides the ultrasound image signals corresponding to the region defined by preset horizontal and vertical lengths. The size of the defined region may vary according to the type of probes, selection of a scanning mode, depth change or the like.

The display unit 140 includes a monitor 141 having a display region 142 configured with dots of an $M \times N$ array, wherein the dots are arrayed in a preset dot pitch. The ultrasound image formed in the image processing unit 120 is displayed on the display region 142. As is well-known, the dot pitch represents a distance P between diagonally neighboring dots. Therefore, a distance between vertically or horizontally neighboring dots (hereinafter referred to as a dot distance) can be calculated by the following equation (1).

$$\text{Dot distance} = \sqrt{\frac{(\text{dot pitch(mm)})^2}{2}} \qquad (1)$$

the real size of the display region 142 having the dots of the $M \times N$ array can be calculated by the following equation (2),

$$\text{Real display region size} = \sqrt{\frac{(\text{dot pitch(mm)})^2}{2}} \cdot \{(M-1)\times(N-1)\}$$

$$= \left\{(M-1)\times\left(\sqrt{\frac{(\text{dot pitch(mm)})^2}{2}}\right)\right\}\times\left\{(N-1)\times\left(\sqrt{\frac{(\text{dot pitch(mm)})^2}{2}}\right)\right\}$$

$$= \{(M-1)\times(N-1)\}\times\left(\sqrt{\frac{(\text{dot pitch(mm)})^2}{2}}\right)^2$$

$$= \{(M-1)\times(N-1)\}\times\frac{\text{dot pitch(mm)}}{2} \qquad (2)$$

For example, when the display region is comprised of $500\times420$ dots and the dot pitch is $0.5mm$, the dot distance becomes $0.35355339mm$ according to the equation (1) and the real display region size becomes $(499\times0.35355339) \times (419\times0.35355339)mm^2$, i.e., $176.42314161\times148.13887041mm^2$. Further, if an ultrasound image, the vertical length of which is $40mm$, is displayed on said display region, the ultrasound image is displayed in a vertical length of about $148.1mm$. That is, the enlarged ultrasound image may be displayed 3.7 times as large as the original length. The operation of the image processing unit 120 for producing an ultrasound image, which displays the target object in real size, will be described below.

The image processing unit 120 forms a first ultrasound image based on the ultrasound image signals received from the ultrasound image signal providing unit 110. The image processing unit 120 adjusts the size of the first ultrasound image so that a second ultrasound image, which displays a target object image in real size, can be formed. As mentioned above, the first ultrasound image formed in the image processing unit 120 is displayed in the preset vertical and horizontal lengths.

The image processing unit 120 calculates a conversion value according to the following equation (3) or (4) and then applies the calculated conversion value to the first ultrasound image, thereby obtaining the second ultrasound image displaying the target object in real size.

$$\text{Conversion value} = \frac{\sqrt{\dfrac{(\text{dot pitch(mm)})^2}{2}} \cdot (M-1)}{\text{vertical length of 1}^{st}\text{ ultrasound image(mm)}} \qquad (3)$$

$$\text{Conversion value} = \frac{\sqrt{\dfrac{(\text{dot pitch(mm)})^2}{2}} \cdot (N-1)}{\text{horizontal length of 1}^{st}\text{ ultrasound image(depth, mm)}} \qquad (4)$$

The user input unit 130 receives an instruction for adjusting the size of the first ultrasound image to the second ultrasound image and then transmits the instruction to the image processing unit 120.

In accordance with one embodiment of the present invention, a distant between diagonally neighboring dots is defined as the dot pitch. However, the dot pitch may be differently defined according to display manufacturers. For example, the dot pitch may represent a distance between horizontally or vertically neighboring dots. In this case, the equation (2) may be expressed as the following equation (5).

$$\text{Real display region size} = \{(M-1) \times (N-1)\} \times (\text{dot pitch})^2 \qquad (5)$$

Also, the equations (3) and (4) may be expressed as the following equations (6) and (7).

$$\text{Conversion value} = \frac{\text{dot pitch} \cdot (M-1)}{\text{vertical length of 1}^{st}\text{ ultrasound image(mm)}} \qquad (6)$$

$$\text{Conversion value} = \frac{\text{dot pitch} \cdot (N-1)}{\text{horizontal length of 1}^{st}\text{ ultrasound image(depth, mm)}} \qquad (7)$$

As mentioned above, the present invention can provide the ultrasound image showing the target object in its real size such that the user can instinctively recognize the size of the target object in the ultrasound image.

According to one aspect of the present invention, there is provided an ultrasound imaging system, comprising: an ultrasound image signal providing unit for providing ultrasound image signals corresponding to an ultrasound image having preset horizontal and vertical lengths; an image processing unit for forming a first ultrasound image based on the ultrasound image signals and adjusting a size of the first ultrasound image to thereby form a second ultrasound image; and a display unit including a display region having a plurality of dots arrayed in horizontal and vertical directions, the display region being configured to display the first and second ultrasound images, wherein the image processing unit calculates a conversion value for enlarging or reducing the first ultrasound image based on a horizontal or vertical length of the first ultrasound image and a distance between the neighboring dots, and wherein the image processing unit applies the conversion value to the first ultrasound image to thereby form the second ultrasound image..

According to another aspect of the present invention, there is provided an ultrasound imaging system, comprising an ultrasound image signal providing unit for providing ultrasound image signals corresponding to an ultrasound image having preset horizontal and vertical lengths; an image processing unit for forming a first ultrasound image based on the ultrasound image signals and adjusting a size of the first ultrasound image to thereby form a second ultrasound image; and a display unit including a display region having a plurality of dots arrayed according to a preset dot pitch, the display region being configured to display the first and second ultrasound images, wherein the image processing unit calculates a distance between horizontally or vertically neighboring dots, a conversion value for enlarging or reducing the first ultrasound image based on a horizontal or vertical length of the first ultrasound image and a distance between the neighboring dots, and wherein the image processing unit applies the conversion value to the first ultrasound image to thereby form the second ultrasound image.

Any reference in this specification to "one embodiment," "an embodiment," "example embodiment," etc., means that a particular feature, structure or characteristic described in connection with the embodiment is included in at least one embodiment of the invention. Such phrases in various places in the specification are not necessarily all referring to the same embodiment. Further, when a particular feature, structure or characteristic is described in connection with any embodiment, it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that fall within the spirit and scope of the principles of this disclosure. More particularly, numerous variations and modifications arc possible in the component parts and/or arrangements of the subject combination arrangement within the scope of the disclosure, the drawings and the appended claims. In addition to the variations and modifications in the component parts and/or arrangements, alternative uses will also be apparent to those skilled in the art.

## Claims

1. An image processing system, comprising:

   an ultrasound image signal providing unit for providing ultrasound image signals corresponding to an ultrasound image having preset horizontal and vertical lengths;
   an image processing unit for forming a first ultrasound image based on the ultrasound image signals and adjusting a size of the first ultrasound image to thereby form a second ultrasound image; and
   a display unit including a display region having a plurality of dots arrayed in horizontal and vertical directions, the display region being configured to display the first and second ultrasound images,

   wherein the image processing unit calculates a conversion value for enlarging or reducing the first ultrasound image based on a horizontal or vertical length of the first ultrasound image and a distance between the neighboring dots, and wherein the image processing unit applies the conversion value to the first ultrasound image to thereby form the second ultrasound image.

2. The ultrasound imaging system of Claim 1, further comprising a user input unit for receiving an instruction for adjusting a size of the first ultrasound image into the second ultrasound image and transmitting the command to the image processing unit

3. An ultrasound imaging system, comprising:

   an ultrasound image signal providing unit for providing ultrasound image signals corresponding to an ultrasound image having preset horizontal and vertical lengths;
   an image processing unit for forming a first ultrasound image based on the ultrasound image signals and adjusting a size of the fust ultrasound image to thereby form a second ultrasound image; and
   a display unit including a display region having a plurality of dots arrayed according to a preset dot pitch, the display region being configured to display the first and second ultrasound images,

   wherein the image processing unit calculates a distance between horizontally or vertically neighboring dots, a conversion value for enlarging or reducing the first ultrasound image based on a horizontal or vertical length of the first ultrasound image and a distance between the neighboring dots, and wherein the image processing unit applies the conversion value to the first ultrasound image to thereby form the second ultrasound image.

4. The ultrasound imaging system of Claim 3, wherein the display region is configured with dots of an $M \times N$ array and the conversion value is calculated by using the following equation:

$$\text{Conversion value} = \frac{\sqrt{\dfrac{(\text{dot pitch(mm)})^2}{2}} \cdot (M-1)}{\text{vertical length of } 1^{\text{st}} \text{ ultrasound image(mm)}}$$

or

$$\text{Conversion value} = \frac{\sqrt{\dfrac{(\text{dot pitch(mm)})^2}{2}} \cdot (M-1)}{\text{horizontal length of } 1^{\text{st}} \text{ ultrasound image(mm)}}$$

5. The ultrasound imaging system of Claim 3, further comprising a user input unit for receiving an instruction for adjusting a size of the first ultrasound image into the second ultrasound image and transmitting the command to the image processing unit.

## FIG. 1

<u>100</u>

110

ULTRASOUND
IMAGE SIGNAL
PROVIDING UNIT

120

IMAGE PROCESSING
UNIT

140

DISPLAY UNIT

USER INPUT UNIT

130

# FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• KR 1020050119852